(19) Europäisches Patentamt
European Patent Office
Office européen des brevets

(11) EP 4 442 673 A1

(12) **EUROPEAN PATENT APPLICATION**
published in accordance with Art. 153(4) EPC

(43) Date of publication:
**09.10.2024 Bulletin 2024/41**

(21) Application number: **22898689.9**

(22) Date of filing: **28.11.2022**

(51) International Patent Classification (IPC):
***C07C 45/50*** *(2006.01)* ***B01J 31/24*** *(2006.01)*
***B01J 31/40*** *(2006.01)* ***C07C 47/02*** *(2006.01)*
***C07B 61/00*** *(2006.01)*

(52) Cooperative Patent Classification (CPC):
(C-Sets available)
**C07C 45/50; B01J 31/24; B01J 31/40;** C07B 61/00

(Cont.)

(86) International application number:
**PCT/JP2022/043774**

(87) International publication number:
**WO 2023/095907 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2021 JP 2021193373**

(71) Applicant: **Mitsubishi Chemical Corporation**
**Tokyo 100-8251 (JP)**

(72) Inventors:
- **MIYAKE, Masashi**
  **Tokyo 100-8251 (JP)**
- **SATO, Takashi**
  **Tokyo 100-8251 (JP)**

(74) Representative: **Hoffmann Eitle**
**Patent- und Rechtsanwälte PartmbB**
**Arabellastraße 30**
**81925 München (DE)**

# (54) METHOD FOR PRODUCING ALDEHYDE

(57) Provided is a method for producing an aldehyde, the method comprising steps (1) to (3) below: (1) a part or the entirety of the reaction solution is extracted from a hydroformylation reaction band while performing the hydroformylation reaction; (2) the extracted reaction solution is brought into contact with an oxygen-containing gas in an atmosphere having a total pressure of 0.8 MPaA or less and an oxygen partial pressure ratio of 10% or less, and is oxidized; and (3) the oxidized reaction solution is supplied to the hydroformylation reaction band while maintaining the state in which the catalyst is dissolved or dispersed in the reaction solution.

FIGURE

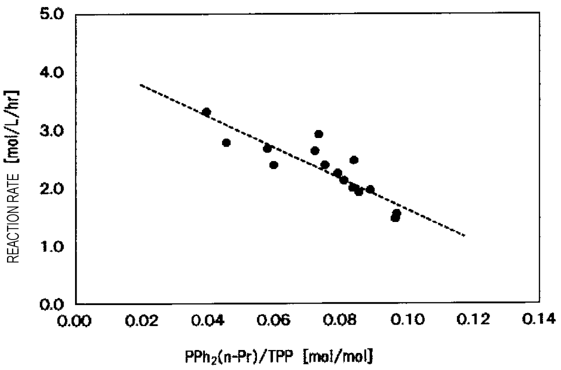


EP 4 442 673 A1

(52) Cooperative Patent Classification (CPC): (Cont.)

C-Sets
**C07C 45/50, C07C 47/02**

**Description**

TECHNICAL FIELD

The present invention relates to a method for producing an aldehyde. More specifically, the present invention relates to a method for producing an aldehyde, in which an aldehyde is produced by subjecting an olefin to a hydroformylation reaction with hydrogen and carbon monoxide in the presence of a catalyst.

BACKGROUND ART

There has been known a method for producing an aldehyde by subjecting an olefin to a hydroformylation reaction with a gas containing hydrogen and carbon monoxide in the presence of a periodic table Group 8 to 10 metal (hereinafter, simply referred to as a "Group 8 to 10 metal")-organophosphorus complex catalyst.

The hydroformylation reaction is also called an "oxo reaction", and a mixed gas of hydrogen ($H_2$) and carbon monoxide (CO) to be used in the reaction is called an "oxo gas".

The catalyst used for the hydroformylation reaction of an olefin contains an expensive Group 8 to 10 metal such as rhodium, and it is therefore ideal to use the catalyst semipermanently. Accordingly, a method in which the reaction product is separated from the reaction solution and the reaction solution containing the catalyst as a distillation residue is fed or circulated to a reaction zone and reused, or a method in which the reaction product is distilled off and separated from a reaction zone by using gas stripping and the reaction is continuously performed while allowing the catalyst-containing reaction solution to remain in the reaction zone, is employed.

However, in the hydroformylation reaction, an inactivated catalyst accumulates in the reaction solution during the reaction. Therefore, a method has been proposed for recovering and reusing such a catalyst.

For example, Patent Document 1 describes a method in which an alkyl phosphine produced by partially substituting a ligand such as triarylphosphine with an alkyl group of α-olefin is treated with an oxygen gas and converted to its corresponding phosphine oxide, and the deactivated catalyst is thereby reactivated.

Patent Document 2 describes a method in which a hydroformylation reaction solution containing a Group 8 metal complex using, as a ligand, a tertiary organophosphorus compound such as triphenylphosphine is put into contact with an oxidizing agent in the presence of a free tertiary organophosphorus compound, a polar organic solvent, water and a basic substance to crystallize and recover a solid complex catalyst of a Group 8 metal.

Patent Document 3 describes a method in which a hydroformylation reaction solution having accumulated therein a high-boiling-point byproduct is mixed with a poor solvent and hydrogen, thereby crystallizing and recovering a hydrogen-coordinated rhodium-phosphine complex catalyst.

Patent Document 4 describes a method in which an alkyl-substituted phosphine produced by partially substituting a ligand such as triphenylphosphine with an alkyl group of α-olefin is subjected to an oxidation treatment, and then mixed with a poor solvent and hydrogen, thereby crystalizing and recovering a hydrogen-coordinated rhodium-phosphine complex catalyst by a crystallization method.

CITATION LIST

PATENT LITERATURE

Patent Literature 1: JPS57-87845A
Patent Literature 2: JPS57-72995A
Patent Literature 3: JP2006-151826A
Patent Literature 4: WO2019/098242

SUMMARY OF INVENTION

TECHNICAL PROBLEM

In the method described in Patent Literature 1, since an activation treatment of an inactivated metal catalyst whose catalytic activity is impaired is performed in the reactor, it is necessary to temporarily stop the hydroformylation reaction, resulting in poor productivity.

In the method described in Patent Literature 2, an operation of recovering the complex catalyst from the crystallized product using a solid-liquid separation method is required, which makes the process complicated. In Patent

Literature 2, the complex catalyst cannot be recovered sufficiently.

In the method described in Patent Literature 3, an operation of recovering the complex catalyst from the crystallized product using a solid-liquid separation method is also required, which makes the process complicated. In Patent Literature 3, the complex catalyst cannot be recovered sufficiently.

In the method described in Patent Literature 4, after an oxidation step, a hydrogen reduction step, a crystallization step, a solid-liquid separation step, and a step of dissolving a solid catalyst recovered by solid-liquid separation in an appropriate solvent and then feeding the solution to a reaction zone are required, which poses problems in terms of a production cost and equipment management. In Patent Literature 4, the complex catalyst cannot be recovered sufficiently.

An object of the present invention is to provide a method for producing an aldehyde capable of recovering, with high efficiency, a highly active complex catalyst from a reaction solution withdrawn outside a reaction zone without stopping a hydroformylation reaction, and capable of reusing the complex catalyst for production of an aldehyde.

## SOLUTION TO PROBLEM

The present inventor has found that by bringing a reaction solution after a hydroformylation reaction into contact with an oxygen-containing gas and feeding an oxidized catalyst solution to a hydroformylation reaction zone, a highly active complex catalyst can be recovered and reused with high efficiency through a simple process in comparison with a method in related art.

The gist of the present invention is as follows.

[1] A method for producing an aldehyde by subjecting an olefin to a hydroformylation reaction with a gas containing hydrogen and carbon monoxide in the presence of a catalyst, the method including the following steps (1) to (3):

(1) withdrawing part or all of a reaction solution from a hydroformylation reaction zone while performing the hydroformylation reaction;
(2) oxidizing by bringing the withdrawn reaction solution into contact with an oxygen-containing gas in an atmosphere having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

[2] A method for producing an aldehyde by subjecting an olefin to a hydroformylation reaction with a gas containing hydrogen and carbon monoxide in the presence of a catalyst, the method including the following steps (1A), (2), and (3):

(1A) withdrawing part or all of a reaction solution from a hydroformylation reaction zone;
(2) oxidizing by bringing the withdrawn reaction solution into contact with an oxygen-containing gas in an atmosphere having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

[3] The method for producing an aldehyde according to [1] or [2], in which
in the step (2), the reaction solution is oxidized by being brought into contact with the oxygen-containing gas in an atmosphere having an oxygen partial pressure of 0.009 MPaA or less.
[4] The method for producing an aldehyde according to any one of [1] to [3], in which
in the step (2), the reaction solution is oxidized such that a ratio of an alkyl-substituted phosphine to an organophosphorus ligand compound in the reaction solution is 0.068 or less.
[5] The method for producing an aldehyde according to any one of [1] to [4], in which
in the step (3), the reaction solution oxidized in the step (2) is fed to the hydroformylation reaction zone while maintaining a non-slurry liquid state.
[6] The method for producing an aldehyde according to any one of [1] to [5], in which
in the step (3), the reaction solution oxidized in the step (2) is subjected to hydrogen reduction in an atmosphere containing carbon monoxide and then fed to the hydroformylation reaction zone.
[7] The method for producing an aldehyde according to any one of [1] to [5], in which
in the step (3), the reaction solution oxidized in the step (2) is fed to the hydroformylation reaction zone, and is first subjected to hydrogen reduction in the reaction zone.
[8] The method for producing an aldehyde according to any one of [1] to [7], in which
the catalyst is a periodic table Group 8 to 10 metal-organophosphorus complex catalyst.
[9] The method for producing an aldehyde according to [8], in which

the periodic table Group 8 to 10 metal is rhodium.

[10] The method for producing an aldehyde according to any one of [1] to [9], in which
in the step (2), in the oxidation, an alkyl-substituted phosphine in the reaction solution is converted to an alkyl-substituted phosphine oxide.

[11] The method for producing an aldehyde according to [10], in which
an oxidation ratio of the alkyl-substituted phosphine is 5.0% to 60.0%.

[12] The method for producing an aldehyde according to any one of [1] to [11], further including:
removing a high-boiling-point byproduct from the reaction solution withdrawn from the hydroformylation reaction zone in the step (1) or the step (1A), and then oxidizing by bringing the reaction solution into contact with the oxygen-containing gas in the step (2).

[13] The method for producing an aldehyde according to any one of [1] to [11], further including:

distilling the reaction solution withdrawn from the hydroformylation reaction zone in the step (1) or the step (1A) to obtain a non-distillate fraction containing a high-boiling-point component containing rhodium; and
oxidizing by subjecting the obtained non-distillate fraction to the step (2) to be brought into contact with the oxygen-containing gas.

[14] The method for producing an aldehyde according to any one of [1] to [11], further including:

distilling, in the step (3), the reaction solution oxidized in the step (2) to obtain a non-distillate fraction containing a obtained high-boiling-point component containing rhodium; and
feeding the obtained non-distillate fraction to the hydroformylation reaction zone.

ADVANTAGEOUS EFFECTS OF INVENTION

According to the present invention, in the method for producing an aldehyde by subjecting an olefin to a hydroformylation reaction with a gas containing hydrogen and carbon monoxide in the presence of a catalyst, a highly active complex catalyst, in particular, an expensive Group 8 to 10 metal in the complex catalyst can be recovered, with high efficiency, from a reaction solution withdrawn outside a reaction zone without stopping the hydroformylation reaction, and can be reused for production of an aldehyde.

According to the present invention, the highly active complex catalyst contained in the reaction solution can be efficiently recovered and reused using relatively simple equipment. As a result, an amount of an expensive Group 8 to 10 transition metal to be used can be reduced, and an increase in production cost can be prevented. Furthermore, a high reaction yield of aldehyde can be maintained during a long-term continuous operation, resulting in excellent productivity.

BRIEF DESCRIPTION OF DRAWINGS

[FIGURE] The FIGURE is a graph showing a relationship between a ratio (a "$PPh_2$(n-Pr)/TPP ratio") of an alkyl-substituted phosphine to an organophosphorus ligand compound and a reaction rate of a recovered catalyst.

DESCRIPTION OF EMBODIMENTS

The present invention is described in detail below. The present invention is not limited to the following description, and can be optionally modified and implemented without departing from the gist of the present invention.

Note that unless otherwise specified, in the present description, a numerical range expressed using "to" means a range that includes numerical values written before and after "to" as an upper limit value and a lower limit value. For example, "A to B" means A or more and B or less.

[Method for Producing Aldehyde]

A first embodiment of a method for producing an aldehyde of the present invention is a method for producing an aldehyde by subjecting an olefin to a hydroformylation reaction with a gas containing hydrogen and carbon monoxide in the presence of a catalyst to be described later, including the following steps (1) to (3):

(1) withdrawing part or all of a reaction solution from a hydroformylation reaction zone while performing the hydroformylation reaction;
(2) oxidizing by bringing the withdrawn reaction solution into contact with an oxygen-containing gas in an atmosphere

having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

In the present description, the "hydroformylation reaction zone" refers to a zone including a reactor for performing a hydroformylation reaction and reactor peripheral equipment such as a gas-liquid separator attached to the reactor
A second embodiment of the method for producing an aldehyde of the present invention is a method for producing an aldehyde by subjecting an olefin to a hydroformylation reaction with a gas containing hydrogen and carbon monoxide in the presence of a catalyst to be described later, including the following steps (1A), (2), and (3):

(1A) withdrawing part or all of a reaction solution from a hydroformylation reaction zone;
(2) oxidizing by bringing the withdrawn reaction solution into contact with an oxygen-containing gas in an atmosphere having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

First, the hydroformylation reaction according to the first and second embodiments of the method for producing an aldehyde of the present invention (hereinafter, these may be collectively referred to as "the present invention") will be described, and then each of the steps (1), (1A), (2), and (3) will be described.

[Hydroformylation Reaction]

[Catalyst]

In the present invention, the catalyst used for the hydroformylation reaction is not particularly limited as long as it has a catalytic effect on the hydroformylation reaction of an olefin. As the catalyst used for the hydroformylation reaction, it is preferable to use a Group 8 to 10 metal-organophosphorus complex catalyst because of excellent reaction activity thereof.
In the present invention, the Group 8 to 10 metal is a metal belongs to Groups 8 to 10 in the periodic table. Among others, ruthenium, cobalt, rhodium, palladium, and platinum are preferred since they have high activity in case of use as a catalyst, and in particular, rhodium is preferably used since it has high activity.
As an organophosphorus ligand compound for forming the Group 8 to 10 metal-organophosphorus complex catalyst, any trivalent organophosphorus compound that commonly functions as a monodentate ligand or a polydentate ligand for the Group 8 to 10 metal can be used. Among them, examples of the organophosphorus compound serving as a monodentate ligand include a third triorganophosphine represented by the following formula [I].

[Chem. 1]

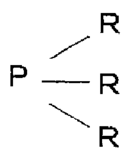

(I)

(In the formula [I], Rs each independently represent a substituted or unsubstituted monovalent hydrocarbon group.)
Examples of the monovalent hydrocarbon group represented by R usually include an alkyl group having 1 to 12 carbon atoms, a cycloalkyl group having 3 to 12 carbon atoms, an aryl group having 3 to 12 carbon atoms, an alkylaryl group having 6 to 24 carbon atoms, and an arylalkyl group having 6 to 24 carbon atoms. That is, examples of the above-described triorganophosphine include trialkylphosphine, triarylphosphine, tricycloalkylphosphine, alkylarylphosphine, cycloalkylarylphosphine, and alkylcycloalkylphosphine.
A substituent that the monovalent hydrocarbon group may have is not particularly limited, and examples thereof include an alkyl group and an alkoxy group.
Specific examples of the triorganophosphine include tributylphosphine, trioctylphosphine, triphenylphosphine, tritolylphosphine, tricycloalkylphosphine, monobutyldiphenylphosphine, dipropylphenylphosphine, and cyclohexyldiphenylphosphine. Among them, triphenylphosphine is preferred because of low activity, chemical stability, and easy availability.
As another example of the trivalent organophosphorus compound, for example, a trivalent phosphite compound

represented by the following formulae (1) to (10) can be used.

<Trivalent Phosphite Compound Represented by Formula (1)>

[Chem. 2]

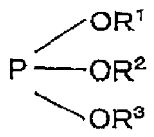

(1)

(In the formula (1), $R^1$ to $R^3$ each independently represent a monovalent hydrocarbon group which may have a substituent.)

Examples of the monovalent hydrocarbon group which may have a substituent and is represented by $R^1$ to $R^3$ include an alkyl group, an aryl group, and a cycloalkyl group.

Specific examples of the compound represented by the formula (1) include trialkyl phosphites such as trimethyl phosphite, triethyl phosphite, n-butyl diethyl phosphite, tri-n-butyl phosphite, tri-n-propyl phosphite, tri-n-octyl phosphite, and tri-n-dodecyl phosphite, triaryl phosphites such as triphenyl phosphite and trinaphthyl phosphite, and alkylaryl phosphites such as dimethylphenyl phosphite, diethylphenyl phosphite, and ethyldiphenyl phosphite. For example, bis(3,6,8-tri-t-butyl-2-naphthyl)phenylphosphite, and bis(3,6,8-tri-t-butyl-2-naphthyl)(4-biphenyl)phenylphosphite described in JP-H6-122642A may be used. Among them, triphenyl phosphite is most preferred.

<Trivalent Phosphite Compound Represented by Formula (2)>

[Chem. 3]

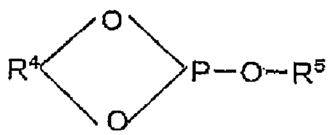

(2)

(In the formula (2), $R^4$ represents a divalent hydrocarbon group which may have a substituent. $R^5$ represents a monovalent hydrocarbon group which may have a substituent.)

Examples of the divalent hydrocarbon group which may have a substituent for $R^4$ include an alkylene group which may contain oxygen, nitrogen, a sulfur atom, etc. in the middle of a carbon chain, a cycloalkylene group which may contain oxygen, nitrogen, a sulfur atom, etc. in the middle of a carbon chain, a divalent aromatic group such as phenylene and naphthylene, a divalent aromatic group to which a divalent aromatic ring is bonded directly or intermediately through an alkylene group or an atom such as oxygen, nitrogen, or sulfur; and a group to which a divalent aromatic group and an alkylene group are bonded directly or intermediately through an atom such as oxygen, nitrogen, or sulfur.

Examples of the monovalent hydrocarbon group for $R^5$ include an alkyl group, an aryl group, and a cycloalkyl group.

Examples of the compound represented by the formula (2) include compounds described in US Patent No. 3415906 specification, such as neopentyl (2,4,6-t-butyl-phenyl) phosphite, and ethylene (2,4,6-t-butyl-phenyl) phosphite.

<Trivalent Phosphite Compound Represented by Formula (3)>

[Chem. 4]

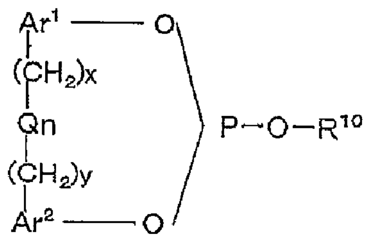

(3)

(In the formula (3), $R^{10}$ has the same meaning as $R^5$ in the above formula (2). $Ar^1$ and $Ar^2$ each independently represent an aryl group which may have a substituent. x and y each independently represent 0 or 1. Q is a crosslinking group selected from the group consisting of $-CR^{11}R^{12}-$, -O-, -S-, $-NR^{13}-$, $-SiR^{14}R^{15}$ and -CO-. $R^{11}$ and $R^{12}$ each independently represent a hydrogen atom, an alkyl group having 1 to 12 carbon atoms, a phenyl group, a tolyl group, or an anisyl group. $R^{13}$, $R^{14}$ and $R^{15}$ each independently represent a hydrogen atom or a methyl group. n represents 0 or 1.)

Specific examples of the trivalent phosphite compound represented by the formula (3) include compounds described in US Patent No. 4599206 specification, such as 1,1'-biphenyl-2,2'-diyl-(2,6-di-t-butyl-4-methylphenyl) phosphite, and compounds described in US Patent No. 4717775 specification, such as 3,3'-di-t-butyl-5,5'-dimethoxy-1,1'-biphenyl-2,2'-diyl(2-t-butyl-4-methoxyphenyl) phosphite.

<Trivalent Phosphite Compound Represented by Formula (4)>

[Chem. 5]

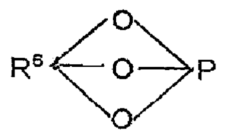

(4)

(In the formula (4), $R^6$ represents a trivalent hydrocarbon group which may have a cyclic or acyclic substituent.)

Examples of the compound represented by the formula (4) include compounds described in US Patent No. 4567306 specification, such as 4-ethyl-2,6,7-trioxa-1-phosphabicyclo-[2,2,2]-octane.

<Trivalent Phosphite Compound Represented by Formulae (5) and (6)>

[Chem. 6]

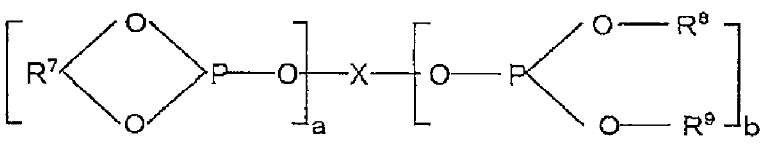

(5)

(In the formula (5), $R^7$ has the same meaning as $R^4$ in the above formula (3). $R^8$ and $R^9$ each independently represent a hydrocarbon group which may have a substituent. a and b each represent an integer of 0 to 6. The sum of a and b is 2 to 6. X represents a (a + b)-valent hydrocarbon group.)

Preferred examples of the compound represented by the formula (5) include a compound represented by the following formula (6). Compounds described in JPS62-116535A and JPS62-116587A are included.

[Chem. 7]

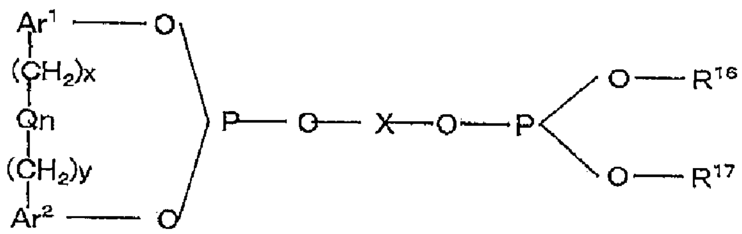

(6)

(In the formula (6), X represents a divalent group selected from the group consisting of alkylene, arylene, and $-Ar^1-(CH_2)x-Qn-(CH_2)y-Ar^2-$. $Ar^1$, $Ar^2$, Q, x, y, and n are the same as $Ar^1$, $Ar^2$, Q, x, y, and n in the above formula (3).)

<Trivalent Phosphite Compound Represented by Formula (7)>

[Chem. 8]

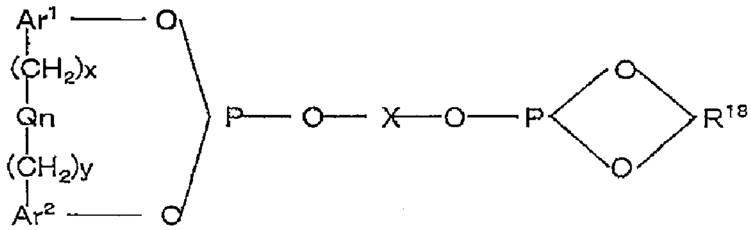

(7)

(In the formula (7), X, $Ar^1$, $Ar^2$, Q, x, y, and n are the same as X, $Ar^1$, $Ar^2$, Q, x, y, and n in the above formula (3). $R^{18}$ has the same meaning as $R^4$ in the above formula (2).)

<Trivalent Phosphite Compound Represented by Formula (8)>

[Chem. 9]

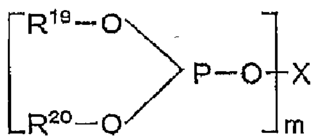

(8)

(In the formula (8), $R^{19}$ and $R^{20}$ each independently represent an aromatic hydrocarbon group, and at least one of the aromatic hydrocarbon groups has a hydrocarbon group at a carbon atom adjacent to a carbon atom to which an oxygen atom is bonded. m represents an integer of 2 to 4. The $-O-P(OR^{19})(OR^{20})$ groups may be different from each other. X represents an m-valent hydrocarbon group which may have a substituent.)

Among the compound represented by the formula (8), for example, compounds described in JPH5-178779A are preferred.

<Trivalent Phosphite Compound Represented by Formula (9)>

[Chem. 10]

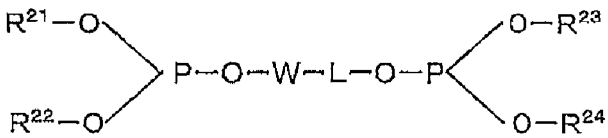

(9)

(In the formula (9), $R^{21}$ to $R^{24}$ each independently represent a hydrocarbon group which may have a substituent. $R^{21}$ and $R^{22}$, and $R^{23}$ and $R^{24}$ may be bonded to each other to form a ring. W represents a divalent aromatic hydrocarbon group which may have a substituent. L represents a saturated or unsaturated divalent aliphatic hydrocarbon group which may have a substituent.)

As the compound represented by the formula (9), for example, those described in JPH8-259578A are used.

<Trivalent Phosphite Compound Represented by Formula (10)>

[Chem. 11]

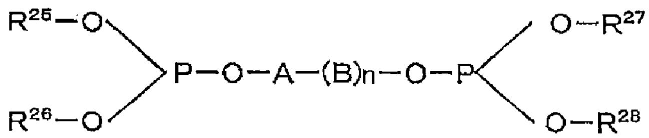

(10)

(In the formula (10), $R^{25}$ to $R^{28}$ each represent a monovalent hydrocarbon group which may have a substituent. $R^{25}$ and $R^{26}$, and $R^{27}$ and $R^{21}$ may be bonded to each other to form a ring. A and B each independently represent a divalent hydrocarbon group which may have a substituent. n represents an integer of 0 or 1.)

Examples of the monovalent hydrocarbon group which may have a substituent and is represented by $R^{25}$ to $R^{21}$ include an alkyl group, an aryl group, and a cycloalkyl group. The divalent hydrocarbon group which may have a substituent for A and B may be any of an aromatic group, an aliphatic group, and an alicyclic group.

These organophosphorus ligand compounds may be used alone or in combination of two or more thereof, and usually are used alone.

From the viewpoint of the oxo reaction, the organophosphorus ligand compound is preferably triorganophosphine represented by the formula (I), and particularly preferably triphenylphosphine.

The Group 8 to 10 metal-organophosphorus complex catalyst can be more easily prepared by a known complex formation method using a periodic table Group 8 to 10 metal compound (hereinafter, referred to as a "Group 8 to 10 metal compound") and an organophosphorus ligand compound. A complex may be formed in the reaction zone by feeding the Group 8 to 10 metal compound and the organophosphorus ligand compound to the reaction zone. In this case, the organophosphorus ligand compound may be directly introduced into the reaction zone but, considering ease of handling, etc., is preferably introduced after dissolving it in a reaction medium.

The Group 8 to 10 metal compound includes, for example, a water-soluble inorganic salt or inorganic complex compound such as rhodium chloride, palladium chloride, ruthenium chloride, platinum chloride, rhodium bromide, rhodium iodide, rhodium sulfate, rhodium nitrate, palladium nitrate, rhodium ammonium chloride and sodium rhodium chloride, and a water-soluble organic acid salt such as rhodium formate, rhodium acetate, palladium acetate, rhodium propionate, palladium propionate and rhodium octanoate. In addition, respective metal complex species may also be used. Among them, in view of excellent reaction activity and catalyst cost, rhodium acetate is preferably used.

[Hydroformylation Reaction Step]

The hydroformylation reaction is performed by reacting an olefin with hydrogen and carbon monoxide in the presence of a catalyst such as a Group 8 to 10 metal-organophosphorus complex catalyst.

Although a carbon number of olefin is not particularly limited, examples thereof include a carbon number of 2 to 20. The olefin having a carbon number of 2 to 20 may be, for example, an $\alpha$-olefin such as ethylene, propylene, 1-butene, 1-pentene, 1-hexene and 1-octene, or an internal olefin such as 2-butene, 2-pentene, 3-hexene and 4-octene.

As the reaction medium for the hydroformylation reaction, a solvent that dissolves a raw material olefin and a catalyst such as a Group 8 to 10 metal-organophosphorus complex catalyst, has a boiling point higher than that of an aldehyde to be produced, and does not inhibit the reaction is preferred. Examples of the solvent that can be used in the hydroformylation reaction include: an aromatic hydrocarbon such as benzene, toluene, and xylene; an aliphatic hydrocarbon such as hexane and octane; esters such as butyl acetate and butyl butyrate ester; and ketones.

A concentration of the catalyst in the reaction medium is usually 1 mass ppm to 10 mass% in terms of metal atom of a Group 8 to 10 metal, etc. The organophosphorus ligand compound such as a phosphine to be used as a ligand is usually present in an excess amount in the reaction medium in order to increase stability of the complex catalyst.

The hydroformylation reaction may be performed under known conditions. For example, when among the Group 8 to 10 metal-organophosphorus complex catalyst, in particular, a rhodium-phosphine complex catalyst is used, the

reaction conditions are usually appropriately selected within the following ranges.

Hydrogen partial pressure: 0.01 MPaG to 20 MPaG
Carbon monoxide partial pressure: 0.01 MPaG to 20 MPaG
Total pressure: 0.02 MPaG to 30 MPaG
Hydrogen partial pressure/carbon monoxide partial pressure: 0.1 to 10
Reaction temperature: 60°C to 200°C
Rh (rhodium) concentration: several mass ppm to several mass%
P (free organophosphorus ligand)/Rh: 2 to 10000 (molar ratio)
Reaction time: several minutes to several tens of hours

In the hydroformylation reaction, an aldehyde having a carbon number of n + 1 can be obtained from a raw material olefin having a carbon number of n (n is, for example, an integer of 2 to 20). Such an aldehyde includes propionaldehyde, butylaldehyde, pentylaldehyde, hexylaldehyde, heptylaldehyde, octylaldehyde, nonylaldehyde, decylaldehyde, etc. Usually, the aldehyde is obtained as a mixture of a linear form and a branched form.
The hydroformylation reaction is performed under the above-described reaction conditions by using usually a flow-type reactor, but a batch-type reactor may also be used.
The main flow reaction (which uses the above flow-type reactor) system includes a stripping system and a liquid circulating system.
The stripping system is a method in which a catalyst-containing reaction solution is held in a reactor, an olefin and an oxo gas are continuously fed, and the aldehyde produced by the reaction is vaporized within the reactor and taken out of the system.
The liquid circulating system is a method in which an olefin, an oxo gas, and a reaction medium containing a catalyst are continuously fed to a reactor and a reaction solution containing the produced aldehyde, the catalyst, the reaction medium, etc. is continuously withdrawn outside the reactor The reaction solution withdrawn from the reactor is separated into the produced aldehyde and a catalyst-containing reaction solution, for example, by a separation operation such as stripping with an unreacted gas or distillation. The obtained produced aldehyde is withdrawn outside the system, and the catalyst-containing reaction solution (in the present invention, the reaction solution corresponds to the reaction solution withdrawn in the step (1) or the step (1A)) is returned to the reactor and recycled.
In the case of the stripping system, a byproduct of hydroformylation reaction which is a high-boiling-point byproduct accumulates in the catalyst-containing reaction solution held within the reactor. Therefore, usually, part of the catalyst-containing reaction solution (in the present invention, the reaction solution corresponds to the reaction solution withdrawn in the step (1) or the step (1A)) is intermittently withdrawn outside the reaction zone.
In the case of the liquid circulating system, when recycling of the catalyst-containing reaction solution is continued, a byproduct which is a high-boiling-point byproduct accumulates in the reaction zone. Therefore, part of the catalyst-containing reaction solution (in the present invention, the reaction solution corresponds to the reaction solution withdrawn in the step (1) or the step (1A)) is continuously or intermittently withdrawn outside the reaction zone.
The amount of the reaction solution withdrawn from the hydroformylation reaction zone, that is, the reactor may be appropriately determined according to the amount of the high-boiling-point byproduct to be produced.
Usually, when the reaction solution is withdrawn outside the reaction zone, a catalyst in an amount corresponding to the catalyst contained in the withdrawn reaction solution are newly fed to the reaction zone. In the present invention, the amount of catalyst to be newly fed can be reduced by subjecting the withdrawn reaction solution to the step (2) and returning the reaction solution to the reaction zone. In particular, for the Group 8 to 10 metal, the reaction can be maintained without replenishment.
The high-boiling-point byproduct is an aldehyde condensate produced by condensation of aldehyde, which is an object product of the hydroformylation reaction.
When a Group 8 to 10 metal-phosphine complex catalyst is used as the catalyst, in the reaction solution having accumulated therein the high-boiling-point byproduct, a phosphine and a phosphine which is alkyl-substituted (hereinafter, a phosphine which is alkyl-substituted is to be referred to as "an alkyl-substituted phosphine") are present.
For example, when rhodium is used as the Group 8 to 10 metal, the following rhodium complexes (a) to (e) are present in the reaction solution having accumulated therein the high-boiling-point byproduct.

(a) a complex in which phosphine is coordinated to rhodium
for example, $RhH(PPh_3)_4$ which is a complex in which triphenylphosphine ($PPh_3$) is coordinated to rhodium (Rh)
(b) a complex in which carbon monoxide and phosphine are coordinated to rhodium for example, $RhH(CO)(PPh_3)_3$
(c) a complex in which carbon monoxide and an alkyl-substituted phosphine are coordinated to rhodium for example, $RhH(CO)(PPh_3)(PPh_2R)_2$ or $RhH(CO)(PPh_2R)_3$ (R represents an alkyl group)
(d) a rhodium cluster complex in which a plurality of rhodiums are connected and carbon monoxide and phosphine

are coordinated thereto

(e) a rhodium cluster complex in which an alkyl-substituted phosphine is coordinated to the rhodium cluster complex above

Among them, a complex in which an alkyl-substituted phosphine is coordinated to rhodium and a rhodium cluster complex exhibit low activity as a complex catalyst. Furthermore, a complex (including a cluster complex), in which an alkyl-substituted phosphine is coordinated, has high solubility in a poor solvent and is less likely to crystallize, in comparison with a complex in which an alkyl-substituted phosphine is not coordinated. In addition, a complex in which an alkyl-substituted phosphine is not coordinated and at least hydrogen and phosphine are coordinated to rhodium has high activity and preferably functions as a complex catalyst for a hydroformylation reaction.

Although rhodium is used as an example in the above, the same can be said for other Group 8 to 10 metals.

[Step (1) and Step (1A)]

The step (1) is a step of withdrawing part or all of a reaction solution from a hydroformylation reaction zone while performing the hydroformylation reaction, as described above.

The step (1A) is a step of withdrawing part or all of a reaction solution from a hydroformylation reaction zone, as described above. The step (1A) is not necessarily required to be performed while the hydroformylation reaction is being performed, and part or all of the reaction solution may be withdrawn from the hydroformylation reaction zone of another hydroformylation reaction series.

Specifically, the step (1) and the step (1A) are a step of withdrawing, outside the reactor, a reaction solution containing an aldehyde produced by the hydroformylation reaction, a catalyst, a reaction medium, etc.

The reaction solution in the hydroformylation reaction zone contains about 40 mass% to 80 mass% of the high-boiling-point byproduct, and by performing the following step (2) on the reaction solution containing the high-boiling-point byproduct, a recovery ratio of the catalyst can be increased in comparison with a method of recovering a catalyst using a solid-liquid separation method after a crystallization treatment in the related art.

Alternatively, in the step (1) or the step (1A), if necessary, the hydroformylation reaction solution withdrawn from the hydroformylation reaction zone may be subjected to the step (2) after a light-boiling-point component in the reaction solution is removed, and oxidized by being brought into contact with the oxygen-containing gas. In order to remove the light-boiling-point component from the reaction solution, known separation operations such as distillation can be used.

Alternatively, in the step (1) or the step (1A), optionally, the hydroformylation reaction solution withdrawn from the hydroformylation reaction zone may be subjected to the step (2) after the high-boiling-point byproduct in the reaction solution is removed, and oxidized by being brought into contact with the oxygen-containing gas. A known separation operation such as distillation can be used to remove the high-boiling-point byproduct from the reaction solution.

Alternatively, in the step (1) or the step (1A), if necessary, the hydroformylation reaction solution withdrawn from the hydroformylation reaction zone may be distilled to obtain a non-distillate fraction containing the high-boiling-point component containing rhodium, and the obtained non-distillate fraction may be subjected to the step (2) and oxidized by being brought into contact with the oxygen-containing gas. A known distillation method can be used for distillation of the hydroformylation reaction solution.

Alternatively, in the step (3), the reaction solution oxidized in the step (2) may be distilled to obtain a non-distillate fraction containing the high-boiling-point component containing rhodium, and the obtained non-distillate fraction may be fed to the hydroformylation reaction zone. A known distillation method can be used for distillation of the hydroformylation reaction solution.

When a rhodium-phosphine complex catalyst is used as the catalyst, the reaction solution withdrawn in the step (1) or the step (1A), that is, the reaction solution before the oxidation treatment usually has the following composition. The reaction solution is subjected to the following step (2).

(Composition of Reaction Solution Withdrawn in Step (1) or Step (1A))

Alkyl-substituted phosphine: 0.5 mass% to 10 mass%
Alkyl-substituted phosphine oxide: 0.1 mass% to 5 mass%
Phosphine: 5 mass% to 50 mass%
Phosphine oxide: 0.5 mass% to 8 mass%
Other components (various complexes, high-boiling-point byproducts, etc.): 40 mass% to 80 mass%

The "various complexes" contained in the "other components" refer to the various rhodium complexes contained in the reaction solution having accumulated therein the high-boiling-point byproduct, as listed in the section of [Hydroformylation Reaction Step] above.

[Step (2)]

The step (2) is a step of oxidizing by bringing the reaction solution withdrawn in the step (1) and the step (1A) into contact with an oxygen-containing gas in an atmosphere having a total pressure of 0.8 MPaA or less and an oxygen partial pressure ratio of 10% or less.

For example, when a Group 8 to 10 metal-phosphine complex is used as the hydroformylation reaction catalyst, it is preferable that, in the step (2), the reaction solution having accumulated therein the high-boiling-point byproduct such as an aldehyde condensation byproduct is oxidized by being brought into contact with an oxygen-containing gas, and the alkyl-substituted phosphine is thereby oxidized and converted to its corresponding alkyl-substituted phosphine oxide. By the conversion, production of an alkyl-substituted phosphine-coordinated complex can be prevented and a decrease in reaction activity as a complex catalyst and a decrease in recovery ratio of a Group 8 to 10 metal-phosphine complex can be prevented.

An alkyl-substituted phosphine has higher compatibility for the Group 8 to 10 metal in comparison with phosphine and tends to be oxidized.

It is preferable that, in the step (2), the oxidation leads to decomposition of a complex in which an alkyl-substituted phosphine is coordinated or a cluster complex. Furthermore, a complex obtained by this decomposition can be recovered as a highly active complex catalyst by feeding the reaction solution after the step (2) to the hydroformylation reaction zone in the step (3).

The reaction solution after the step (2), that is, the reaction solution after the oxidation treatment usually has the following composition.

(Composition of Reaction Solution After Step (2))

Alkyl-substituted phosphine: 0.1 mass% to 6 mass%
Alkyl-substituted phosphine oxide: 0.1 mass% to 10 mass%
Phosphine: 5 mass% to 40 mass%
Phosphine oxide: 1 mass% to 10 mass%
Other components (various complexes, high-boiling-point byproducts, etc.): 40 mass% to 80 mass%

When rhodium is used as the Group 8 to 10 metal, examples of the highly active complex catalyst include $RhH(CO)(PPh_3)_3$ and $RhH(PPh_3)_4$.

Preferred examples of the oxygen-containing gas to be used in the step (2) include oxygen, air, and a gas obtained by adding an inert gas such as nitrogen to air.

A total pressure in the step (2) is 0.8 MPaA or less, and the oxygen partial pressure ratio is 10% or less.

An upper limit of the total pressure in the step (2) is 0.8 MPaA or less since oxidation of phosphine can be prevented and an activity ratio of the recovered Group 8 to 10 metal-phosphine complex catalyst increases. The total pressure is preferably 0.6 MPaA or less, and more preferably 0.4 MPaA or less.

On the other hand, a lower limit of the total pressure is not particularly limited, but when the total pressure is excessively low, the amount of an alkyl-substituted phosphine-coordinated complex in the reaction solution cannot be sufficiently reduced, and the activity ratio of the recovered Group 8 to 10 metal-phosphine complex catalyst decreases. Therefore, usually, the total pressure is preferably 0.01 MPaA or more, more preferably 0.02 MPaA or more, and still more preferably 0.05 MPaA or more.

The upper limit and the lower limit can be freely combined. That is, the total pressure in the step (2) is preferably 0.01 MPaA to 0.8 MPaA, more preferably 0.02 MPaA to 0.6 MPaA, and still more preferably 0.0.05 MPaA to 0.4 MPaA.

An upper limit of the oxygen partial pressure ratio in the step (2) is 10% or less since oxidation of phosphine can be prevented and the activity ratio of the recovered Group 8 to 10 metal-phosphine complex catalyst increases. The oxygen partial pressure ratio is more preferably 9% or less, and still more preferably 8% or less. On the other hand, a lower limit of the oxygen partial pressure ratio is not particularly limited, but when the oxygen partial pressure ratio is excessively low, the amount of an alkyl-substituted phosphine-coordinated complex in the reaction solution cannot be sufficiently reduced, and the activity ratio of the recovered Group 8 to 10 metal-phosphine complex catalyst decreases. Therefore, usually, the oxygen partial pressure ratio is preferably 2% or more, and particularly preferably 4% or more.

The upper limit and the lower limit can be freely combined. That is, the oxygen partial pressure ratio in the step

(2) is preferably 2% to 10%, more preferably 4% to 9%, and still more preferably 4% to 8%.

In the step (2), an upper limit of the oxygen partial pressure is not particularly limited, and is preferably 0.009 MPaA or less, more preferably 0.008 MPaA or less, and still more preferably 0.007 MPaA or less since the total pressure and the oxygen partial pressure ratio are satisfied, oxidation of phosphine can be prevented, and the activity ratio of the recovered Group 8 to 10 metal-phosphine complex catalyst increases. On the other hand, a lower limit of the oxygen partial pressure is not particularly limited, and when the oxygen partial pressure is excessively low, the amount of an alkyl-substituted phosphine-coordinated complex in the reaction solution cannot be sufficiently reduced, and the activity ratio of the recovered Group 8 to 10 metal-phosphine complex catalyst decreases. Therefore, usually, the oxygen partial pressure is preferably 0.001 MPaA or more, more preferably 0.002 MPaA or more, and still more preferably 0.004 MPaA or more.

The upper limit and the lower limit can be freely combined. That is, the oxygen partial pressure in the step (2) is preferably 0.001 to 0.009, more preferably 0.002 to 0.008, and still more preferably 0.004 to 0.007.

In the step (2), an upper limit of a ratio of the alkyl-substituted phosphine to the organophosphorus ligand compound (hereinafter, may be referred to as an "alkyl-substituted phosphine/phosphine ratio") in the withdrawn reaction solution is not particularly limited, and the ratio is preferably 0.068 or less, more preferably 0.060 or less, still more preferably 0.058 or less, and particularly preferably 0.052 or less since oxidation of phosphine can be prevented and the activity ratio of the recovered Group 8 to 10 metal-phosphine complex catalyst increases. On the other hand, a lower limit of the alkyl-substituted phosphine/phosphine ratio is not particularly limited, and the alkyl-substituted phosphine/phosphine ratio is preferably 0.010 or more, more preferably 0.015 or more, still more preferably 0.020 or more, and particularly preferably 0.025 or more since the amount of an alkyl-substituted phosphine-coordinated complex in the reaction solution is reduced and the activity ratio of the recovered Group 8 to 10 metal-phosphine complex catalyst can be satisfactorily maintained.

The upper limit and the lower limit can be freely combined. That is, the alkyl-substituted phosphine/phosphine ratio is preferably 0.010 or more and 0.068 or less, more preferably 0.015 or more and 0.060 or less, still more preferably 0.020 or more and 0.058 or less, and particularly preferably 0.025 or more and 0.052 or less.

Examples of the method for performing the oxidation treatment such that the alkyl-substituted phosphine/phosphine ratio falls within the above range include a method of adjusting known conditions such as an oxygen concentration, an oxygen partial pressure, an oxidation time, and an oxidation temperature when the reaction solution is withdrawn from the reaction zone and then the withdrawn reaction solution is oxidized by being brought into contact with an oxygen-containing gas.

When the Group 8 to 10 metal-phosphine complex is used as the hydroformylation reaction catalyst, an oxidation ratio of the alkyl-substituted phosphine in the step (2) is preferably 5.0% to 60.0%, more preferably 10.0% to 55.0%, and still more preferably 15.0% to 50.0%.

It is preferable that the oxidation ratio is higher than a lower limit of the above ratio range, since the amount of an alkyl-substituted phosphine-coordinated complex in the reaction solution decreases, and the recovery ratio of the Group 8 to 10 metal-phosphine complex catalyst increases. In addition, it is preferable that the oxidation ratio is lower than the upper limit of the above ratio range, since oxidation of phosphine can be prevented and the amount of phosphine reused in the reaction zone does not decrease.

Here, the oxidation ratio (%) of the alkyl-substituted phosphine is represented by the following formula.

Oxidation ratio(%) = {(amount of alkyl-substituted phosphine in reaction solution before oxidation treatment - amount of alkyl-substituted phosphine in reaction solution after oxidation treatment)/amount of alkyl-substituted phosphine in reaction solution before oxidation treatment} $\times$ 100

The change in the amount of an alkyl-substituted phosphine, etc. between before and after oxidation can be easily detected by a conventional analysis method such as gas chromatography.

Examples of the method for performing the oxidation treatment such that the oxidation ratio of the alkyl-substituted phosphine falls within the above range include a method of adjusting known conditions such as an oxygen concentration, an oxygen partial pressure, an oxidation time, and an oxidation temperature when the reaction solution is withdrawn from the reaction zone and then the withdrawn reaction solution is oxidized by being brought into contact with an oxygen-containing gas.

The oxidation treatment in the step (2) is preferably performed at a temperature of 85°C to 180°C, more preferably 90°C to 180°C, further more preferably 110°C to 180°C, particularly preferably 110°C to 160°C, and most preferably 110°C to 150°C.

It is preferable that the oxidation treatment temperature is higher than the lower limit of the above range, since conversion of the alkyl-substituted phosphine to its corresponding alkyl-substituted phosphine oxide is sufficient, and the recovery ratio of the Group 8 to 10 metal-phosphine complex catalyst further increases. It is preferable that the

temperature is lower than the upper limit of the above range, since oxidation of phosphine can be prevented, and the amount of phosphine reused in the reaction zone does not decrease.

When the oxidation treatment temperature is 110°C to 150°C, decomposition of the cluster complex is more promoted and the highly active Group 8 to 10 metal complex such as a rhodium complex increases.

The oxidation treatment time in the step (2) varies depending on other conditions such as temperature but, usually, is approximately several minutes to several hours, and specifically, 1 hour to 5 hours is preferable.

The specific treatment method in the step (2) is not particularly limited, and in order to implement the total pressure, the oxygen partial pressure and the ratio thereof, and the oxidation treatment temperature as described above, examples thereof include a method in which a continuous stirring tank type reactor equipped with a jacket and a stirring blade is usually used, and the reaction solution withdrawn in the step (1) or the step (1A) and the oxygen-containing gas are continuously fed thereto, and the oxidation treatment is performed using the stirring blade at a predetermined temperature for a predetermined residence time.

[Step (3)]

In the step (3), the reaction solution subjected to the oxidation treatment in the step (2) is fed to the hydroformylation reaction zone while maintaining a state in which the catalyst is dissolved or dispersed in the reaction solution.

Here, "while maintaining a state in which the catalyst is dissolved or dispersed in the reaction solution" means that the reaction solution after the oxidation treatment that is fed to the hydroformylation reaction zone is a catalyst-containing liquid. In the step (3), it is preferable that the reaction solution subjected to the oxidation treatment in the step (2) is fed to the hydroformylation reaction zone while maintaining a non-slurry liquid state, in other words, a solution state in which the catalyst is not precipitated.

When the reaction solution after the oxidation treatment that is to be fed to the hydroformylation reaction zone in the step (3) is in a slurry form, that is, a slurry containing a crystallized product and a mother liquid, the crystallized product is lost when a catalyst-containing crystallized product in the reaction solution is recovered using a known separation method such as a solid-liquid separation method, and thus the recovery ratio of the catalyst is insufficient. Therefore, in the present invention, by feeding or circulating the reaction solution in a non-slurry state, preferably in a solution state, into the hydroformylation reaction zone, the loss of the catalyst-containing crystallized product can be eliminated, and the recovery ratio of the catalyst can be increased.

In order to feed or circulate the reaction solution in a non-slurry state, preferably in a solution state, into the hydroformylation reaction zone, a reaction solution in a non-slurry state, preferably in a solution state, that is obtained by subjecting the reaction solution to the oxidation treatment under a specific oxidation condition may be fed or circulated into the hydroformylation reaction zone without forming the reaction solution into a slurry state using a crystallization method requiring a solid-liquid separation method.

In the step (3), optionally, the reaction solution oxidized in the step (2) may be fed to the hydroformylation reaction zone after hydrogen reduction in an atmosphere containing carbon monoxide. In the step (3), optionally, the reaction solution oxidized in the step (2) may be directly fed to the hydroformylation reaction zone, and hydrogen reduction may be performed for a first time in the reaction zone.

That is, in the reaction solution oxidized in the step (2), the alkyl-substituted phosphine is converted into its corresponding alkyl-substituted phosphine oxide by the oxidation, but part of the alkyl-substituted phosphine is not oxidized and remains as a complex in which an alkyl-substituted phosphine is coordinated to rhodium or a rhodium cluster complex, and these complexes do not exhibit high catalytic activity as the hydroformylation reaction catalyst.

Therefore, in the step (3), optionally, before the reaction solution oxidized in the step (2) is fed to the hydroformylation reaction zone, the complex in which an alkyl-substituted phosphine is coordinated to rhodium or the rhodium cluster complex may be hydrogen-reduced in an atmosphere containing carbon monoxide to enhance the catalytic activity and then fed to the hydroformylation reaction zone.

In the step (3), the hydrogen reduction in an atmosphere containing carbon monoxide can be performed under known conditions. For example, when a rhodium-phosphine complex catalyst is used, the reaction conditions for the hydrogen reduction are usually selected as appropriate within the following range.

Hydrogen partial pressure: 0.01 MPaG to 20 MPaG
Carbon monoxide partial pressure: 0.01 MPaG to 20 MPaG
Total pressure: 0.02 MPaG to 30 MPaG
Hydrogen partial pressure/carbon monoxide partial pressure: 0.1 to 10
Reaction temperature: 60°C to 200°C
Reaction time: several minutes to several tens of hours

Since the hydroformylation reaction zone is fed with hydrogen and carbon monoxide to create a reducing

atmosphere, the reaction solution oxidized in step (2) is directly fed to the hydroformylation reaction zone without separately performing the hydrogen reduction as described above, and is hydrogen-reduced within the reaction zone. Therefore, the hydrogen reduction treatment of the reaction solution is not necessarily required.

In the present invention, after the reaction solution withdrawn in the step (1) or the step (1A) is subjected to the oxidation treatment in the step (2), in the step (3), the reaction solution subjected to the oxidation treatment is fed to the hydroformylation reaction zone without performing operations such as crystallization, with the catalyst component in the oxidation reaction solution contained in the solution as it is. Therefore, the catalyst component, in particular, the Group 8 to 10 metal such as expensive rhodium, can be reused in the hydroformylation reaction at a recovery ratio of substantially 100% without loss.

In the present invention, after the reaction solution is continuously withdrawn in the step (1) or the step (1A) and the withdrawn reaction solution is continuously subjected to the oxidation treatment in the step (2), in the step (3), the reaction solution subjected to the oxidation treatment may be circulated and fed to the hydroformylation reaction zone without performing operations such as crystallization, with the catalyst component in the oxidation reaction solution contained in the solution as it is.

The activity ratio of the catalyst in the oxidation reaction solution to be fed to the hydroformylation reaction zone in the step (3) can be determined by comparing a hydroformylation reaction rate A in a hydroformylation reaction performed using the catalyst, and a reaction rate B of the same hydroformylation reaction performed using a new catalyst in the same manner.

Specifically, the activity ratio (%) is represented by the following formula.

$$\text{Activity ratio (\%)} = (\text{reaction rate A/reaction rate B}) \times 100$$

The above activity ratio can also be determined by comparing the reaction rate of the catalyst between immediately before performing the oxidation treatment in the step (2) and immediately after the processing. The reaction rate can be observed, for example, as a decrease rate of the raw material olefin, carbon monoxide or hydrogen.

According to the present invention, the reaction solution withdrawn from the hydroformylation reaction zone is subjected to the oxidation treatment under predetermined conditions and then fed to the hydroformylation reaction zone, whereby the recovery ratio and the activity ratio of the catalyst can be increased in comparison with the case where the oxidation treatment is performed under conditions that deviate from the predetermined conditions.

According to the present invention, the recovery ratio of the catalyst can also be increased in comparison with the case where the catalyst is crystallized after the oxidation treatment.

## Examples

Hereinafter, the present invention will be described in more detail with reference to Examples. The present invention is not limited to the following Examples as long as the scope of the present invention is not exceeded.

(Oxidation Ratio of Alkyl-substituted Phosphine)

An oxidation ratio of an alkyl-substituted phosphine was calculated by the following formula. In the present Example, n-propyldiphenylphosphine was used as the alkyl-substituted phosphine.

[Formula 1]

$$\text{Oxidation ratio (unit: \%) of alkyl-substituted phosphine} = \frac{\left(\text{Content ratio (unit: mass\%) of alkyl-substituted phosphine in reaction solution before oxidation treatment} - \text{Content ratio (unit: mass\%) of alkyl-substituted phosphine in reaction solution after oxidation treatment}\right)}{\left(\text{Content ratio (unit: mass\%) of alkyl-substituted phosphine in reaction solution before oxidation treatment}\right)} \times 100$$

(Oxidation Ratio of Organophosphorus Ligand Compound)

An oxidation ratio of an organophosphorus ligand compound was calculated using the following formula. In the present Example, measurement targets were triphenylphosphine as the organophosphorus ligand compound and triphenylphosphine oxide, which was converted from triphenylphosphine by oxidation.

[Formula 2]

$$\text{Oxidation ratio (unit: \%) of organophosphorus ligand compound} = \frac{\left(\text{Content ratio (unit: mass\%) of triphenylphosphine oxide in reaction solution after oxidation treatment} - \text{Content ratio (unit: mass\%) of triphenylphosphine oxide in reaction solution before oxidation treatment}\right)}{\left(\text{Molecular weight (unit: g/mol) of triphenylphosphine oxide}\right)} \times \frac{\left(\text{Molecular weight (unit: g/mol) of triphenylphosphine}\right)}{\left(\text{Content ratio (unit: mass\%) of triphenylphosphine in reaction solution before oxidation treatment}\right)} \times 100$$

(Ratio of Oxidation Ratio)

As an index indicating regeneration efficiency of a catalyst contained in a reaction solution after a hydroformylation reaction, a ratio of oxidation ratio (1)/(2) was calculated based on an oxidation ratio (referred to as an "oxidation ratio (1)") of the alkyl-substituted phosphine (n-propyldiphenylphosphine) and an oxidation ratio (referred to as an "oxidation ratio (2)") of the organophosphorus ligand compound (triphenylphosphine), and evaluated according to the following criteria.

$$\text{Ratio of oxidation ratio (1)/(2)} = \text{oxidation ratio (1)/oxidation ratio (2)}$$

A: Ratio of oxidation ratio (1)/(2) is 7.0 or more
B: Ratio of oxidation ratio (1)/(2) is 5.5 or more and less than 7.0
C: Ratio of oxidation ratio (1)/(2) is less than 5.5

It can be seen that as the value of the ratio of oxidation ratio (1)/(2) is larger, oxidation and decomposition of the alkyl-substituted phosphine (n-propyldiphenylphosphine) tends to be promoted, and oxidation and decomposition of the organophosphorus ligand compound (triphenylphosphine) tend to be prevented. That is, the larger the value of the ratio of oxidation ratio (1)/(2), the more efficiently the inactivated catalyst contained in the reaction solution after the hydroformylation reaction was regenerated.

(Rh Recovery Ratio)

An amount of rhodium (in terms of rhodium atom) in the reaction solution before an oxidation treatment and a treated product (the reaction solution after the oxidation treatment or a crystallized product after crystallization in the case of performing the crystallization) was measured by a fluorescent X-ray fluorescence analysis method, and a rhodium recovery ratio was calculated using the following formula, and evaluated according to the following criteria.

Rh revovery ratio (%) = amount (unit: mg) of rhodium in treated product/amount (unit: mg) of rhodium in reaction solution before oxidation treatment $\times$ 100

A: Rh recovery ratio is 90% to 100%
B: Rh recovery ratio is 80% or more and less than 90%
C: Rh recovery ratio is less than 80%

[Example 1]

A hydroformylation reaction of propylene was performed using rhodium acetate as a Group 8 to 10 metal compound and triphenylphosphine as an organophosphorus ligand compound. Next, a reaction solution was withdrawn from a hydroformylation reaction zone, and a light-boiling-point component was distilled off using a distillation method. A composition of the reaction solution after distillation and before being subjected to an oxidation treatment was as follows.
A solution composition was determined by a gas chromatography internal standard method.

(Reaction Solution Composition Before Oxidation Treatment)

n-Propyldiphenylphosphine: 1.12 mass%
n-Propyldiphenylphosphine oxide: 0.46 mass%
Triphenylphosphine: 21.17 mass%
Triphenylphosphine oxide: 1.31 mass%
Other components (various complexes, high-boiling-point byproducts, etc.): 75.94 mass%

Next, the withdrawn reaction solution was continuously fed to a continuous stirring tank type reactor (volume: 250 mL) equipped with a jacket and a stirring blade at a flow rate of 6.0 mL/min with air and a nitrogen ($N_2$) gas containing 6.0 vol% of oxygen at an air feed rate of 0.09 L/min and an $N_2$ gas feed rate of 0.21 L/min, and the oxidation treatment was performed under oxidation conditions shown in Table 1 while stirring using the stirring blade at 500 rpm.
The solution composition (after oxidation) of the reaction solution after the oxidation treatment was analyzed, and as shown in Table 1, the oxidation ratio of n-propyldiphenylphosphine was 21.6 mass%, and the oxidation ratio of triphenylphosphine was 2.3 mass%. Since the reaction solution after the oxidation treatment was amber and transparent, it was confirmed that the catalyst was dissolved in the reaction solution and a cluster complex was decomposed.
Since there is no rhodium withdrawn outside the hydroformylation reaction zone after the oxidation treatment, a rhodium recovery ratio in Example 1 is 100% in terms of rhodium atom.
In the present Example, the "oxidation temperature" is a fluid temperature in the reactor The "oxidation time" is a residence time of the reaction solution in the reactor, which is calculated based on an amount of liquid present in the reactor and a solution feed rate.

[Comparative Example 1]

A raw material solution having the following solution composition was continuously fed to a continuous stirring tank type reactor (volume: 342 mL) equipped with a jacket and a stirring blade at a flow rate of 6.0 mL/min with a nitrogen gas containing 6 vol% of oxygen at a flow rate of 0.35 L/min, followed by stirring at 500 rpm using the stirring blade.

(Raw Material Solution Composition Before Oxidation Treatment)

n-Propyldiphenylphosphine: 1.22 mass%
n-Propyldiphenylphosphine oxide: 0.47 mass%
Triphenylphosphine: 17.44 mass%
Triphenylphosphine oxide: 1.06 mass%
Other components (various complexes, high-boiling-point byproducts, etc.): 79.81 mass%

Next, the reaction solution was continuously withdrawn from the reactor, and an oxidation treatment was performed under the same conditions as in Example 1 except that the oxidation conditions shown in Table 1 were used. The solution composition of the reaction solution after the oxidation treatment was shown in Table 1.
Next, a hydroformylation reaction was performed by continuously feeding, to a continuous stirring tank type reactor (volume: 250 mL) equipped with a jacket and a stirring blade, a mixed gas of hydrogen and carbon monoxide (hydrogen:carbon monoxide = 1:1 (volume ratio)) at 35 NL/hr, propylene at 34.7 g/hr, and the reaction solution after the oxidation treatment at a flow rate of 325 g/hr under reaction conditions of a reaction temperature of 100°C, a reaction pressure of 2.0 MPaG, and a stirring rotation speed of 630 rpm. The reaction solution was withdrawn from the continuous stirring tank type reactor, a light-boiling-point component was distilled off using a distillation method, and then the reaction solution was returned to the above-described continuous stirring tank type reactor.

In Comparative Example 1, the "oxidation time" is a value obtained by dividing an amount of liquid present in the reactor for performing the oxidation treatment by a solution feed rate, and is an integrated value of the residence time of the reaction solution in the reactor

An oxidation ratio of n-propyldiphenylphosphine and an oxidation ratio of triphenylphosphine were measured using gas chromatography for the reaction solution after the oxidation treatment after an oxidation time of 7.44 hrs. The results were shown in Table 1.

Furthermore, 80 (g) of the reaction solution after the oxidation treatment and 331 (g) of a mixed solvent of isopropyl alcohol and water (isopropyl alcohol:water = 65:35 (weight ratio)) were put in an autoclave of an electromagnetic induction stirrer having a volume of 0.5 L in an inert gas atmosphere. After tightly closing the autoclave, a hydrogen gas was injected at a temperature of 15°C to reach a pressure of 0.9 MPaG while stirring at 600 rpm, and the system was held at the pressure and temperature for 2 hours to precipitate a complex catalyst. Thereafter, the hydrogen gas was purged, and solid-liquid separation was performed by normal vacuum filtration.

The amount of the separated rhodium complex was quantified, and the recovery ratio of rhodium complex was determined. As a result, the recovery ratio was 82.1 mass% in terms of rhodium atom.

[Comparative Example 2]

As shown in Table 1, an oxation reaction and crystallization were performed under the same conditions as in Comparative Example 1, except that a solution composition of a raw material solution and a time of an oxidation crystallization treatment were shortened. An oxidation ratio of n-propyldiphenylphosphine and an oxidation ratio of triphenylphosphine were measured using gas chromatography for the reaction solution after the oxidation treatment after an oxidation time of 0.93 hrs. The results were shown in Table 1.

The amount of the rhodium complex separated by crystallization was quantified, and the recovery ratio of rhodium complex was determined. As a result, the recovery ratio was 72.9 mass% in terms of rhodium atom.

[Comparative Example 3]

Under the same conditions as in Example 1, hydroformylation of propylene was performed using rhodium acetate as a Group 8 to 10 metal compound and triphenylphosphine as a phosphine ligand. Next, a reaction solution was withdrawn from a hydroformylation reaction zone, and a light-boiling-point component was distilled off using a distillation method. A composition of the reaction solution after distillation and before being subjected to an oxidation treatment was as follows.

A solution composition was determined by a gas chromatography internal standard method.

(Solution Composition Before Oxidation Treatment)

n-Propyldiphenylphosphine: 1.21 mass%
n-Propyldiphenylphosphine oxide: 0.56 mass%
Triphenylphosphine: 27.08 mass%
Triphenylphosphine oxide: 1.49 mass%
Other components (various complexes, high-boiling-point byproducts, etc.): 69.66 mass%

The withdrawn reaction solution was fed in an amount of 10 L to a complete mixing reactor (volume: 20 L) equipped with a jacket, air and a nitrogen ($N_2$) gas containing 6 vol% of oxygen were fed at different feed rates shown in Table 1, and the oxidation treatment was performed for 0.5 hours while stirring at 500 rpm using a stirring blade.

A solution composition of the reaction solution after the oxidation treatment and various evaluation results were shown in Table 1.

[Table 1]

Table 1

| | | | Example 1 | | Comparative Example 1 | |
|---|---|---|---|---|---|---|
| Oxidation conditions | Oxidation temperature | °C | 150 | | 150 | |
| | Oxidation time | hr | 0.63 | | 7.44 | |
| | Air feed rate | L/min | 0.09 | | 0.35 | |
| | Nitrogen gas feed rate | L/min | 0.21 | | | |
| | Oxygen concentration in fed gas | vol% | 6.0 | | 6.0 | |
| | Total pressure | MPaA | 0.10 | | 0.10 | |
| | Oxygen partial pressure | MPaA | 0.0060 | | 0.0060 | |
| | Oxygen partial pressure ratio | % | 6.0 | | 6.0 | |
| | Solution feed rate | mL/min | 6.0 | | 6.0 | |
| | [n-Propyldiphenylphosphine] / [triphenylphosphine] ratio | mol/mol | 0.050 | | 0.053 | |
| Crystallization | | | No | | Yes | |
| Solution composition | | | Before oxidation | After oxidation | Before oxidation | After oxidation |
| | n-Propyldiphenylphosphine | mass% | 1.12 | 0.88 | 1.22 | 0.62 |
| | n-Propyldiphenylphosphine oxide | mass% | 0.46 | 0.64 | 0.47 | 0.94 |
| | Triphenylphosphine | mass% | 21.17 | 20.26 | 17.44 | 13.37 |
| | Triphenylphosphine oxide | mass% | 1.31 | 1.82 | 1.06 | 3.19 |
| | Others (various complexes, high-boiling-point byproducts, etc.) | mass% | 75.94 | 76.40 | 79.81 | 81.88 |
| Results | n-Propyldiphenylphosphine oxidation ratio (1) | % | 21.6 | | 49.4 | |
| | Triphenylphosphine oxidation ratio (2) | % | 2.3 | | 11.5 | |
| | Ratio of oxidation ratio (1)/(2) | - | 9.6 | | 4.3 | |
| | | Determination | A | | C | |
| | Rh recovery ratio | % | 100 | | 82.1 | |
| | | Determination | A | | B | |

Table 1 (continued)

| | | | Comparative Example 2 | | Comparative Example 3 | |
|---|---|---|---|---|---|---|
| Oxidation conditions | Oxidation temperature | °C | 150 | | 150 | |
| | Oxidation time | hr | 0.93 | | 0.50 | |
| | Air feed rate | L/min | 0.35 | | 3.01 | |
| | Nitrogen gas feed rate | L/min | | | 3.01 | |
| | Oxygen concentration in fed gas | vol% | 6.0 | | 10.2 | |
| | Total pressure | MPaA | 0.10 | | 0.10 | |
| | Oxygen partial pressure | MPaA | 0.0060 | | 0.0102 | |
| | Oxygen partial pressure ratio | % | 6.0 | | 10.2 | |
| | Solution feed rate | mL/min | 6.0 | | - | |
| | [n-Propyldiphenylphosphine] / [triphenylphosphine] ratio | mol/mol | 0.069 | | 0.042 | |
| Crystallization | | | Yes | | No | |
| Solution composition | | | Before oxidation | After oxidation | Before oxidation | After oxidation |
| | n-Propyldiphenylphosphine | mass% | 1.22 | 1.01 | 1.21 | 0.92 |
| | n-Propyldiphenylphosphine oxide | mass% | 0.47 | 0.68 | 0.56 | 0.77 |
| | Triphenylphosphine | mass% | 17.44 | 16.77 | 27.08 | 24.96 |
| | Triphenylphosphine oxide | mass% | 1.06 | 1.56 | 1.49 | 2.47 |
| | Others (various complexes, high-boiling-point byproducts, etc.) | mass% | 79.81 | 79.98 | 69.66 | 70.88 |
| Results | n-Propyldiphenylphosphine oxidation ratio (1) | % | 17.5 | | 24.0 | |
| | Triphenylphosphine oxidation ratio (2) | % | 2.7 | | 3.4 | |
| | Ratio of oxidation ratio (1)/(2) | - | 6.5 | | 7.03 | |
| | | Determination | B | | B | |
| | Rh recovery ratio | % | 72.9 | | 100 | |
| | | Determination | C | | A | |

[Examples 2 to 8]

An oxidation reaction and an oxidation treatment were performed under the same conditions as in Example 1, except that a temperature of the oxidation treatment, a flow rate of a fed gas, and a residence time of a reaction solution in the reactor were changed as shown in Table 2. In all of Examples 2 to 8, since the reaction solution after the oxidation treatment was amber and transparent, it was confirmed that the catalyst was dissolved in the reaction solution.

The results of Examples 2 to 8 were shown in Table 2.

[Table 2]

Table 2

| | | | Example 2 | | Example 3 | | Example 4 | |
|---|---|---|---|---|---|---|---|---|
| Oxidation conditions | Oxidation temperature | °C | 130 | | 140 | | 150 | |
| | Oxidation time | hr | 0.65 | | 0.64 | | 0.62 | |
| | Air feed rate | L/min | 0.09 | | 0.09 | | 0.17 | |
| | Nitrogen feed rate | L/min | 0.21 | | 0.21 | | 0.43 | |
| | Oxygen concentration in fed gas | vol% | 6.0 | | 6.0 | | 6.0 | |
| | Total pressure | MPaA | 0.10 | | 0.10 | | 0.10 | |
| | Oxygen partial pressure | MPaA | 0.0060 | | 0.0060 | | 0.0060 | |
| | Oxygen partial pressure ratio | % | 6.0 | | 6.0 | | 6.0 | |
| | Solution feed rate | mL/min | 6.0 | | 6.0 | | 6.0 | |
| | [n-Propyldiphenylphosphine]/ [triphenylphosphine] ratio | mol/mol | 0.049 | | 0.049 | | 0.050 | |
| Crystallization | | | No | | No | | No | |
| Solution composition | | | Before oxidation | After oxidation | Before oxidation | After oxidation | Before oxidation | After oxidation |
| | n-Propyldiphenylphosphine | mass% | 1.10 | 0.87 | 1.12 | 0.88 | 1.17 | 0.75 |
| | n-Propyldiphenylphosphine oxide | mass% | 0.44 | 0.66 | 0.45 | 0.64 | 0.50 | 0.81 |
| | Triphenylphosphine | mass% | 21.35 | 20.59 | 21.45 | 20.53 | 19.20 | 17.22 |
| | Triphenylphosphine oxide | mass% | 1.29 | 1.85 | 1.34 | 1.84 | 1.42 | 2.24 |
| | Others (various complexes, high-boiling-point byproducts, etc.) | mass% | 75.83 | 76.02 | 75.64 | 76.12 | 77.70 | 78.98 |

(continued)

| | | | Example 2 | Example 3 | Example 4 |
|---|---|---|---|---|---|
| Results | n-Propyldiphenylphosphine oxidation ratio (1) | % | 20.4 | 21.8 | 36.4 |
| | Triphenylphosphine oxidation ratio (2) | % | 2.5 | 2.2 | 4.0 |
| | Ratio of oxidation ratio (1)/(2) | - | 8.2 | 9.9 | 9.0 |
| | | Determination | A | A | A |
| | Rh recovery ratio | % | 100 | 100 | 100 |
| | | Determination | A | A | A |

Table 2 (continued)

| | | | Example 5 | | Example 6 | | Example 7 | | Example 8 | |
|---|---|---|---|---|---|---|---|---|---|---|
| Oxidation conditions | Oxidation temperature | °C | 150 | | 150 | | 150 | | 150 | |
| | Oxidation time | hr | 0.61 | | 0.60 | | 0.42 | | 1.25 | |
| | Air feed rate | L/min | 0.35 | | 0.44 | | 0.09 | | 0.09 | |
| | Nitrogen feed rate | L/min | 0.87 | | 1.09 | | 0.21 | | 0.21 | |
| | Oxygen concentration in fed gas | vol% | 6.0 | | 6.0 | | 6.0 | | 6.0 | |
| | Total pressure | MPaA | 0.10 | | 0.10 | | 0.10 | | 0.10 | |
| | Oxygen partial pressure | MPaA | 0.0060 | | 0.0060 | | 0.0060 | | 0.0060 | |
| | Oxygen partial pressure ratio | % | 6.0 | | 6.0 | | 6.0 | | 6.0 | |
| | Solution feed rate | mL/min | 6.0 | | 6.0 | | 9.0 | | 3.0 | |
| | [n-Propyldiphenylphosphine]/[triphenylphosphine] ratio | mol/mol | 0.038 | | 0.046 | | 0.053 | | 0.042 | |
| Crystallization | | | No | | No | | No | | No | |
| Solution composition | | | Before oxidation | After oxidation | Before oxidation | After oxidation | Before oxidation | After oxidation | Before oxidation | After oxidation |
| | n-Propyldiphenylphosphine | mass% | 1.11 | 0.62 | 1.34 | 0.71 | 1.11 | 0.94 | 1.11 | 0.69 |
| | n-Propyldiphenylphosphine oxide | mass% | 0.47 | 0.91 | 0.55 | 1.04 | 0.46 | 0.62 | 0.46 | 0.82 |
| | Triphenylphosphine | mass% | 19.99 | 18.65 | 20.00 | 17.84 | 20.96 | 20.21 | 21.03 | 19.20 |
| | Triphenylphosphine oxide | mass% | 1.35 | 2.61 | 1.19 | 2.19 | 1.32 | 1.74 | 1.34 | 2.36 |
| | Others (various complexes, high-boiling-point byproducts, etc.) | mass% | 77.09 | 77.22 | 76.92 | 78.23 | 76.15 | 76.49 | 76.06 | 76.92 |

(continued)

| | | | Example 5 | Example 6 | Example 7 | Example 8 |
|---|---|---|---|---|---|---|
| Results | n-Propyldiphenylphosphine oxidation ratio (1) | % | 44.4 | 46.9 | 15.4 | 37.5 |
| | Triphenylphosphine oxidation ratio (2) | % | 5.9 | 4.7 | 1.9 | 4.6 |
| | Ratio of oxidation ratio (1)/(2) | - | 7.5 | 10.0 | 8.0 | 8.2 |
| | | Determination | A | A | A | A |
| | Rh recovery ratio | % | 100 | 100 | 100 | 100 |
| | | Determination | A | A | A | A |

Under the conditions in Examples 1 to 8, a ratio (a ratio of oxidation ratio (1)/(2)) of an alkyl-substituted phosphine oxidation ratio to an organophosphorus ligand compound oxidation ratio and a rhodium recovery ratio were high. That is, the inactivated catalyst contained in the reaction solution after the hydroformylation reaction could be efficiently regenerated, and the highly active complex catalyst could be recovered with high efficiency.

On the other hand, under the conditions in Comparative Examples 1 and 2, since the crystallization treatment was performed without performing the oxidation treatment of the present invention, the rhodium recovery ratio was insufficient.

Under the conditions in Comparative Example 3, since the oxygen partial pressure ratio was high, the ratio (the ratio of oxidation ratio (1)/(2)) of the alkyl-substituted phosphine oxidation ratio to the organophosphorus ligand compound oxidation ratio and the rhodium recovery ratio was low and insufficient. That is, the regeneration efficiency of the inactivated catalyst contained in the reaction solution after the hydroformylation reaction and the activity of the recovered complex catalyst were insufficient.

[Reference Example 1 and Experimental Examples 1 to 16]

In order to examine a relationship between a ratio (a "$PPh_2$(n-Pr)/TPP ratio") of alkyl-substituted phosphine to an organophosphorus ligand compound after the oxidation treatment and the activity of the recovered catalyst, the following experiment was performed.

(Production of Aldehyde)

A hydroformylation reaction was performed by continuously feeding, to a continuous stirring tank type reactor (volume: 250 mL) equipped with a jacket and a stirring blade, a mixed gas of hydrogen and carbon monoxide (hydrogen:carbon monoxide = 1: 1 (volume ratio)) at 35 NL/hr, propylene at 34.7 g/hr, and the catalyst-containing reaction solution after the oxidation treatment described in Reference Example 1 in Table 3 at a flow rate of 325 g/hr under reaction conditions of a reaction temperature of 100°C, a reaction pressure of 2.0 MPaG, and a stirring rotation speed of 630 rpm.

A solution composition of the reaction solution sent out from an outlet of the continuous stirring tank type reactor before the hydroformylation reaction (Reference Example 1) and after the hydroformylation reaction (Experimental Examples 1 to 16) was analyzed. The results were shown in Table 3.

In the present Reference Example and the present Experimental Example, since an aldehyde is not contained in the solution before the hydroformylation reaction, an "aldehyde concentration" is a concentration (unit: mass%) of aldehyde produced by the hydroformylation reaction.

The "residence time" is a time obtained by dividing an outlet flow rate by a reactor volume.

The "reaction rate" is a production rate (unit: mol/L/hr) of aldehyde. The aldehyde was determined by a gas chromatography internal standard method.

The "$PPh_2$ (n-Pr)/TPP ratio" is a ratio of the alkyl-substituted phosphine to the organophosphorus ligand compound in the reaction solution at the outlet of the reactor In the present experiment, the ratio is a molar ratio of n-propyldiphenylphosphine to triphenylphosphine. Each compound was determined by a gas chromatography internal standard method.

[Table 3]

| | Aldehyde | $PPh_2$(n-Pr) | TPP | Flow rate at reactor outlet | Residence time in reactor | $PPh_2$(n-Pr)/ TPP ratio | Reaction rate |
|---|---|---|---|---|---|---|---|
| | mass% | mass% | mass% | g/hr | hr | mol/mol | mol/L/hr |
| Reference Example 1 (before reaction) | - | 1.22 | 17.44 | - | - | - | - |
| Experimental Example 1 | 5.59 | 1.37 | 16.35 | 344 | 0.73 | 0.096 | 1.47 |
| Experimental Example 2 | 5.85 | 1.38 | 16.35 | 345 | 0.72 | 0.097 | 1.55 |
| Experimental Example 3 | 5.60 | 1.30 | 15.57 | 344 | 0.73 | 0.096 | 1.47 |

(continued)

| | Aldehyde | $PPh_2$(n-Pr) | TPP | Flow rate at reactor outlet | Residence time in reactor | $PPh_2$(n-Pr)/ TPP ratio | Reaction rate |
|---|---|---|---|---|---|---|---|
| | mass% | mass% | mass% | g/hr | hr | mol/mol | mol/L/hr |
| Experimental Example 4 | 7.21 | 1.21 | 15.70 | 350 | 0.71 | 0.089 | 1.96 |
| Experimental Example 5 | 7.07 | 1.10 | 14.87 | 350 | 0.71 | 0.085 | 1.92 |
| Experimental Example 6 | 7.35 | 1.10 | 15.24 | 351 | 0.71 | 0.083 | 2.01 |
| Experimental Example 7 | 7.76 | 1.06 | 15.15 | 352 | 0.71 | 0.081 | 2.14 |
| Experimental Example 8 | 8.76 | 1.08 | 14.81 | 356 | 0.70 | 0.084 | 2.47 |
| Experimental Example 9 | 8.12 | 1.03 | 15.01 | 354 | 0.71 | 0.079 | 2.25 |
| Experimental Example 10 | 8.52 | 0.97 | 14.92 | 355 | 0.70 | 0.075 | 2.38 |
| Experimental Example 11 | 10.07 | 0.98 | 15.41 | 361 | 0.69 | 0.073 | 2.92 |
| Experimental Example 12 | 9.29 | 0.95 | 15.15 | 358 | 0.70 | 0.072 | 2.65 |
| Experimental Example 13 | 8.53 | 0.70 | 13.48 | 355 | 0.70 | 0.060 | 2.39 |
| Experimental Example 14 | 9.37 | 0.70 | 13.84 | 359 | 0.70 | 0.058 | 2.67 |
| Experimental Example 15 | 9.70 | 0.56 | 14.08 | 360 | 0.69 | 0.046 | 2.79 |
| Experimental Example 16 | 11.15 | 0.49 | 14.07 | 366 | 0.68 | 0.040 | 3.31 |

Next, the relationship between the ratio (the "$PPh_2$(n-Pr)/TPP ratio") of the alkyl-substituted phosphine to the organophosphorus ligand compound shown in Table 3 and the reaction rate of the recovered catalyst was plotted in the FIGURE.

It is understood from the FIGURE that the reaction rate of the recovered catalyst, that is, the activity of the recovered catalyst tends to increase as the value of the ratio (the "$PPh_2$ (n-Pr)/TPP ratio") of the alkyl-substituted phosphine to the organophosphorus ligand compound after the oxidation treatment decreases.

Although the present invention has been explained in detail using specific embodiments, it is obvious to those skilled in the art that various modifications can be made without departing from the spirit and the scope of the present invention.

The present application is based on a Japanese patent application filed on November 29, 2021 (patent application No. 2021-193373), which is hereby incorporated by reference in its entirety.

## Claims

1. A method for producing an aldehyde by subjecting an olefin to a hydroformylation reaction with a gas containing hydrogen and carbon monoxide in the presence of a catalyst, the method comprising the following steps (1) to (3):

(1) withdrawing part or all of a reaction solution from a hydroformylation reaction zone while performing the hydroformylation reaction;
(2) oxidizing by bringing the withdrawn reaction solution into contact with an oxygen-containing gas in an atmosphere having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

**2.** A method for producing an aldehyde by subjecting an olefin to a hydroformylation reaction with a gas containing hydrogen and carbon monoxide in the presence of a catalyst, the method comprising the following steps (1A), (2), and (3):

(1A) withdrawing part or all of a reaction solution from a hydroformylation reaction zone;
(2) oxidizing by bringing the withdrawn reaction solution into contact with an oxygen-containing gas in an atmosphere having a total pressure of 0.8 MPaA or less and an oxygen partial pressure ratio of 10% or less; and
(3) feeding the oxidized reaction solution to the hydroformylation reaction zone while maintaining a state in which the catalyst is dissolved or dispersed in the reaction solution.

**3.** The method for producing an aldehyde according to claim 1 or 2, wherein
in the step (2), the reaction solution is oxidized by being brought into contact with the oxygen-containing gas in an atmosphere having an oxygen partial pressure of 0.009 MPaA or less.

**4.** The method for producing an aldehyde according to any one of claims 1 to 3, wherein
in the step (2), the reaction solution is oxidized such that a ratio of an alkyl-substituted phosphine to an organophosphorus ligand compound in the reaction solution is 0.068 or less.

**5.** The method for producing an aldehyde according to any one of claims 1 to 4, wherein
in the step (3), the reaction solution oxidized in the step (2) is fed to the hydroformylation reaction zone while maintaining a non-slurry liquid state.

**6.** The method for producing an aldehyde according to any one of claims 1 to 5, wherein
in the step (3), the reaction solution oxidized in the step (2) is subjected to hydrogen reduction in an atmosphere containing carbon monoxide and then fed to the hydroformylation reaction zone.

**7.** The method for producing an aldehyde according to any one of claims 1 to 5, wherein
in the step (3), the reaction solution oxidized in the step (2) is fed to the hydroformylation reaction zone, and is first subjected to hydrogen reduction in the reaction zone.

**8.** The method for producing an aldehyde according to any one of claims 1 to 7, wherein
the catalyst is a periodic table Group 8 to 10 metal-organophosphorus complex catalyst.

**9.** The method for producing an aldehyde according to claim 8, wherein
the periodic table Group 8 to 10 metal is rhodium.

**10.** The method for producing an aldehyde according to any one of claims 1 to 9, wherein
in the step (2), in the oxidation, an alkyl-substituted phosphine in the reaction solution is converted to an alkyl-substituted phosphine oxide.

**11.** The method for producing an aldehyde according to claim 10, wherein
an oxidation ratio of the alkyl-substituted phosphine is 5.0% to 60.0%.

**12.** The method for producing an aldehyde according to any one of claims 1 to 11, further comprising:
removing a high-boiling-point byproduct from the reaction solution withdrawn from the hydroformylation reaction zone in the step (1) or the step (1A), and then oxidizing by bringing the reaction solution into contact with the oxygen-containing gas in the step (2).

**13.** The method for producing an aldehyde according to any one of claims 1 to 11, further comprising:

distilling the reaction solution withdrawn from the hydroformylation reaction zone in the step (1) or the step (1A)

to obtain a non-distillate fraction containing a high-boiling-point component containing rhodium; and
oxidizing by subjecting the obtained non-distillate fraction to the step (2) to be brought into contact with the oxygen-containing gas.

**14.** The method for producing an aldehyde according to any one of claims 1 to 11, further comprising:

distilling, in the step (3), the reaction solution oxidized in the step (2) to obtain a non-distillate fraction containing a obtained high-boiling-point component containing rhodium; and
feeding the obtained non-distillate fraction to the hydroformylation reaction zone.

FIGURE

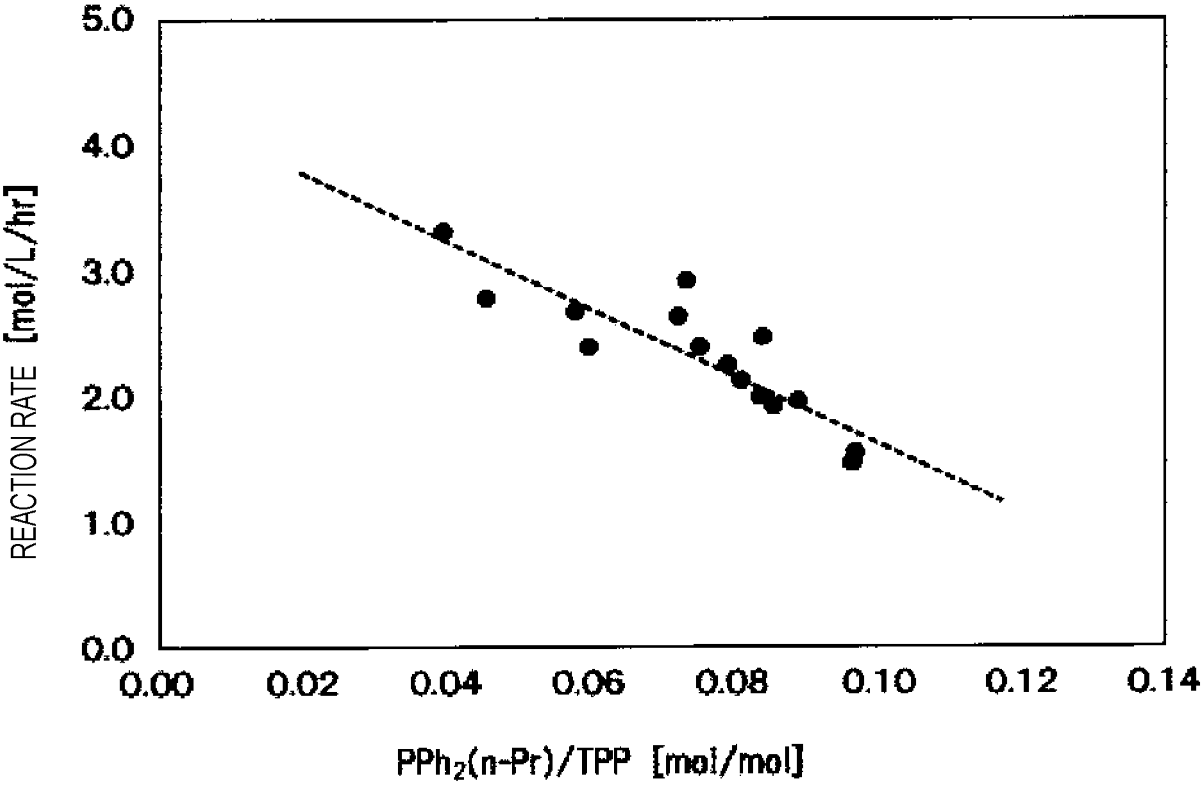
5.0
4.0
3.0
2.0
1.0
0.0
REACTION RATE [mol/L/hr]
0.00
0.02
0.04
0.06
0.08
0.10
0.12
0.14
PPh2(n-Pr)/TPP [mol/mol]

**INTERNATIONAL SEARCH REPORT**

International application No. **PCT/JP2022/043774**

**A. CLASSIFICATION OF SUBJECT MATTER**

***C07C 45/50***(2006.01)i; ***B01J 31/24***(2006.01)i; ***B01J 31/40***(2006.01)i; ***C07C 47/02***(2006.01)i; *C07B 61/00*(2006.01)n
FI: C07C45/50; C07C47/02; B01J31/40 Z; B01J31/24 Z; C07B61/00 300

According to International Patent Classification (IPC) or to both national classification and IPC

**B. FIELDS SEARCHED**

Minimum documentation searched (classification system followed by classification symbols)

C07C45/50; B01J31/24; B01J31/40; C07C47/02; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus (STN)

**C. DOCUMENTS CONSIDERED TO BE RELEVANT**

| Category* | Citation of document, with indication, where appropriate, of the relevant passages | Relevant to claim No. |
|---|---|---|
| X | JP 51-023212 A (MITSUBISHI CHEM IND) 24 February 1976 (1976-02-24) claims, examples, p. 2, lower left column, p. 3, lower left column, p. 3, lower right column | 1-3, 5, 7-9, 13-14 |
| Y | claims, examples, p. 2, lower left column, p. 3, lower left column, p. 3, lower right column | 4, 6, 10-12 |
| Y | WO 2019/098242 A1 (MITSUBISHI CHEM CORP) 23 May 2019 (2019-05-23) claims, paragraphs [0028]-[0040] | 4, 10-12 |
| Y | JP 59-109245 A (DAICEL KAGAKU KOGYO KK) 23 June 1984 (1984-06-23) p. 3, lower left column | 6 |
| A | US 5290743 A (ARCO CHEMICAL TECHNOLOGY L.P.) 01 March 1994 (1994-03-01) claims, examples | 1-14 |
| A | JP 57-087845 A (UNION CARBIDE CORP) 01 June 1982 (1982-06-01) claims, examples | 1-14 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 February 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/043774**

| Patent document cited in search report | Publication date (day/month/year) | Patent family member(s) | Publication date (day/month/year) |
|---|---|---|---|
| JP 51-023212 A | 24 February 1976 | (Family: none) | |
| WO 2019/098242 A1 | 23 May 2019 | BR 112020009474 A2 | |
| | | US 2020/0270186 A1 claims, paragraphs [0045]-[0071] | |
| | | CN 111344273 A | |
| | | EP 3712126 A1 | |
| | | RU 2020115799 A | |
| JP 59-109245 A | 23 June 1984 | NL 8304295 A | |
| | | US 4537997 A column 4, lines 46-55 | |
| US 5290743 A | 01 March 1994 | (Family: none) | |
| JP 57-087845 A | 01 June 1982 | AT 6992 T | |
| | | US 4605780 A claims, examples | |
| | | BR 8105868 A | |
| | | CA 1187100 A | |
| | | EP 49781 A1 | |
| | | ES 505725 A1 | |
| | | KR 10-1988-0000058 B1 | |
| | | MX 158918 A | |
| | | PL 233146 A1 | |
| | | SU 1757458 A3 | |
| | | YU 228081 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S5787845 A
- JP S5772995 A
- JP 2006151826 A
- WO 2019098242 A
- JP H6122642 A
- US 3415906 A
- US 4599206 A
- US 4717775 A
- US 4567306 A
- JP S62116535 A
- JP S62116587 A
- JP H5178779 A
- JP H8259578 A
- JP 2021193373 A